# EUROPEAN PATENT SPECIFICATION

(11) **EP 3 655 424 B1**
(45) Date of publication and mention of the grant of the patent: **13.10.2021**
(21) Application number: 18755225.2
(22) Date of filing: 19.07.2018
(51) Int. Cl.: C07K 14/435, A61K 38/00, A61P 15/00

(54) **PEPTIDES MODULATING SPERM MOTILITY BY MODULATING THE PHOSPHOPROTEIN PHOSPHATASE 1 COMPLEX.**
PEPTIDE, DIE DIE SPERMIENMOTILITÄT DURCH MODULATION DES PHOSPHOPROTEIN-PHOSPHATASE-1-KOMPLEXES MODULIEREN
PEPTIDES MODULANT LA MOTILITÉ DU SPERME PAR LA MODULATION DU COMPLEXE DE PHOSPHOPROTÉINE PHOSPHATASE 1

(30) Priority: 19.07.2017 GB 201711620
(43) Date of publication of application: 27.05.2020
(73) Proprietor: University of Wolverhampton, Wolverhampton, West Midlands WV1 1LY (GB); Universidade de Aveiro, Aveiro 3810-193 (PT)
(72) Inventor: FARDILHA, Margarida Sâncio da Cruz, Aveiro 3800-123 (PT); VIEIRA DA SILVA, Joana, São João da Madeira 3700-069 (PT); FREITAS, Maria João, 9350-142 Ribeira Brava Madeira (PT); HOWL, John, Womboune South Staffordshire WV5 8AL (GB); JONES, Sarah, Wolverhampton West Midlands WV4 5AA (GB)
(74) Representative: Barker Brettell LLP
(86) International application number: PCT/GB2018/052046
(87) International publication number: WO 2019/016555

(56) References cited:
- WO-A2-97/03660
- JOANA VIEIRA SILVA ET AL: "Construction and analysis of a human testis/sperm-enriched interaction network: Unraveling the PPP1CC2 interactome", BIOCHIMICA ET BIOPHYSICA ACTA (BBA) - GENERAL SUBJECTS, vol. 1861, no. 2, 1 February 2017 (2017-02-01), pages 375-385, XP055518592, AMSTERDAM, NL ISSN: 0304-4165, DOI: 10.1016/j.bbagen.2016.11.041
- DATABASE Geneseq [Online] 18 November 2004 (2004-11-18), "Tumour-associated antigenic target (TAT) polypeptide PRO82243, SEQ:3799.", XP002786143, retrieved from EBI accession no. GSP:ABM81473 Database accession no. ABM81473 & WO 2004/030615 A2 (GENENTECH INC [US]; WU THOMAS D [US]; ZHANG ZEMIN [US]; ZHOU YAN [US]) 15 April 2004 (2004-04-15)
- HENDRICKX A ET AL: "Docking Motif-Guided Mapping of the Interactome of Protein Phosphatase-1", CHEMISTRY AND BIOLOGY, CURRENT BIOLOGY, LONDON, GB, vol. 16, no. 4, 24 April 2009 (2009-04-24) , pages 365-371, XP026046187, ISSN: 1074-5521, DOI: 10.1016/J.CHEMBIOL.2009.02.012 [retrieved on 2009-04-23]
- JONES, S. ET AL.: "Intracellular translocation and differential accumulation of cell penetrating peptides in bovine spermatozoa: Evaluation of efficient delivery vectors that do not compromise human sperm motility", HUM. REPROD., vol. 28, 2013, pages 1874-89, XP55518873, cited in the application

## Description

The present invention relates to novel peptides and the uses thereof, in particular for use in male contraception or for use in the treatment of male infertility.

Male infertility is a troubling medical condition with social and economical influences for couples who desire pregnancy. Spermatozoa acquire motility and fertility in the epididymal lumen which is a complex microenvironment. Phosphorylation, glycosylation and further processing are several of the post-translational modifications that sperm proteins undergo during epididymal transit, resulting in changes in protein function, ultimately leading to mature spermatozoa.

Sperm function, in particular sperm motility remains an important parameter when investigating male infertility with one of the causes of male infertility being attributed to compromised sperm motility such as non-progressive sperm motility. There is therefore a need to develop agents able to modulate sperm motility, and preferably to improve sperm motility, for use in the treatment of male infertility

Although many clinical trials on hormone based male contraceptives have been conducted, there still remains a lack of male contraception, particularly with respect to non-invasive contraceptive methods. The currently available contraceptive options for males include condoms, vasectomy and withdrawal which are not ideal. One approach to address the need for more available male contraception would be to modulate sperm motility. It would therefore be desirable to have a method of specifically modulating sperm motility without affecting the hormonal control of sperm production in the testis which could be used as a contraceptive. Preferably this would have the advantages of no side effects, a problem with hormonal therapies, as well as being reversible.

Mature spermatozoa do not translate their haploid genome and lack many conventional cellular processes that could be considered as feasible therapeutic targets. The unique plasma membrane that surrounds mature spermatozoa, coupled with a lack of endocytotic pathways, is also a major caveat to their therapeutic manipulation employing conventional agents which are generally impermeable. Hence it is important that studies like J V Silva et al [8] are performed. Furthermore, it should be noted that WO 2004/030615 A2 discloses a protein which represents a tumour-associated antigen target (TAT), and that Jones S et al., 2013[3] discloses intracellular translocation and differential accumulation of cell penetrating peptides in bovine spermatozoa and evaluates different delivery vectors that allegedly do not compromise human sperm fertility.

There is therefore a need to develop novel therapies and methods for treating male infertility and/or to provide novel male contraception aimed at modulating sperm function, in particular sperm motility.

An aim of the present invention is to provide novel peptides which can be used as a male contraceptive and/or also for the treatment of male infertility where sperm motility is compromised.

As used herein, increased sperm motility refers to a statistically-verified increase in the number of motile sperm within a given sample. In human clinical samples, increased sperm motility may also be measured by a change in the class of sperm from non-motile to non-progressively motile and/or from non-progressively motile to progressively motile.

Sperm motility may be increased by about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or more.

As used herein, decreased sperm motility refers to a statistically-verified decrease in the number of non-motile sperm within a given sample. In human clinical samples, decreased sperm motility may also be measured by a change in the class of sperm from progressively motile to non-progressively motile and/or non-progressively motile to non-motile.

Sperm motility may be decreased by about 0.25 fold to about 0.5 fold, about 0.5 fold to about 0.75 fold, about 0.75 fold to about 1.0 fold, or about 1.0 fold to about 1.5 fold.

According to one aspect of the invention, there is provided a peptide according to the first or second aspect for use in increasing or decreasing sperm motility by modulation of the phosphoprotein phosphatase 1 complexes.

The disclosure, which does not form part of the invention, relates to a peptide for use in increasing or decreasing sperm motility by modulation of PPP1 complexes, wherein the peptide comprises or consists of the following amino acid sequence:
*H*-GQQDQDR**KVICF**VAVSTLNV-*NH₂* (AKAP4-BM)

In this nomenclature *H*- indicates a proton added to NH to form NH₂ at the amino terminal, whereas *-NH₂* indicates that the peptide is amidated at the C-terminal.

The disclosure, which does not form part of the invention, relates to a peptide for use in increasing or decreasing sperm motility by modulation of PPP1 complexes, wherein the peptide comprises or consists of the following amino acid sequence:
*H*-KPNATRPVTPPRVASGLNPSIQKASNYRNNTVLY-*NH₂* (PPP1CC2-CT)

According to a first aspect of the invention, there is provided a peptide comprising or consisting of the following amino acid sequence:
*H*-GQQDQDR**KVICF**VAVSTLNV*-NH₂*

According to a second aspect of the invention, there is provided a peptide comprising or consisting of the following amino acid sequence:
*H*-KPNATRPVTPPRVASGLNPSIQKASNYRNNTVLY*-NH₂*

A 'peptide' as used herein refers to a sequence of amino acids made up of a single chain of amino acids joined by peptide bonds. Generally, peptides contain at least 2 amino acid residues and are less than 50 amino acids in length unless otherwise defined. A peptide may refer to a protein-protein modulator peptide which is also known as a bioportide. A peptide is able to modulate the activity of phosphoprotein phosphatase 1 by blocking the interactions which keep the phosphoprotein phosphatase 1 active.

All peptide sequences mentioned herein are written according to the usual convention whereby the N-terminal amino acid is on the left and the C-terminal amino acid is on the right. Unless otherwise indicated, where the amino acid has isomeric forms, it is the L form of the amino acid that is represented.

The peptide according to the invention may be able to translocate into the flagellum of the spermatozoa.

The peptide may be modified by substitution, insertion or deletion of one or more amino acids, but still have substantially the same activity or function as the unmodified peptide sequence.

The peptide may include a "conservative substitution" in which an amino acid is substituted for another amino acid that has similar properties. Amino acid substitutions may generally be made on the basis of similarity in polarity, charge, solubility, hydrophobicity, hydrophilicity and/or the amphipathic nature of the residues. For example, negatively charged amino acids include aspartic acid and glutamic acid; positively charged amino acids include lysine and arginine; and amino acids with uncharged polar head groups having similar hydrophilicity values include leucine, isoleucine, and valine; glycine, and alanine; asparagine and glutamine; and serine, threonine, phenylalanine, and tyrosine. Other groups of amino acids that may represent conservative changes include (1) Ala, Pro, Gly, glu, Asp, Asn, Ser, Thr; (2) Cys, Ser, Tyr, Thr; (3) Val, Ile, Leu, Met, Ala, Phe; (4) Lys, Arg,Hhis; and (5) Phe, Tyr, Trp, His.

Sequences can also be modified by common structural changes to amino- and/or carboxyl termini, the substitution of non-coded and/or D-enantiomer amino acids and topographical changes resulting from full or partial retro, inverso and retro-inverso transformations of the primary sequence^{[6,10]}.

When describing conserved sequence motifs, including PIP binding site motifs, the symbol 'X' is used for a position where any amino acid is accepted. Ambiguities are indicated by listing the acceptable amino acids for a given position between square brackets '[]'. For example: [WLT] stands for Trp or Leu or Thr. Additional ambiguities are also indicated by listing between a pair of curly brackets '{ }' the amino acids that are not accepted at a given position. For example: {AM} stands for all any amino acid except Ala and Met. Each element in a pattern is separated from its neighbour by a '-'. Repetition of an element of the pattern can be indicated by following that element with a numerical value or, if it is a gap ('X'), by a numerical range between parentheses. For examples: X(3) would correspond to X-X-X whilst M(2,4) would corresponds to M-M, M-M-M or M-M-M-M.

A peptide according to the invention may comprise a cell-penetrating peptide. Preferably, the peptide is linked or conjugated to a cell-penetrating peptide by a chemical bond that can be a peptide bond, a disulphide bond or other form of conjugation.

A cell-penetrating peptide (CPP) is a peptide, usually 10-30 amino acids in length, which is able to penetrate cellular membranes for example to translocate different cargoes into cells^{[4]}.

A bioportide is a CPP that has intrinsic bioactivity following cellular internalization^{[5,6,9]}. Bioportides described herein (PPP1-modulatory bioportides) are designed to change the activity of Phosphoprotein Phosphatase 1 (PPP1) complexes by blocking or mimicking the interactions of PPP1 with PPP1-interacting proteins (PIPs) that regulate its activity.

The cell-penetrating peptide may translocate the peptide into the flagellum of the spermatozoa.

The cell penetrating peptide may be selected from the group consisting of penetratin (RQIKIWFQNRRMKWKK), HIV-derived tat (GRKKRRQRRRPQRNYF), transportan 10 (AGYLLGKINLKALAALAKKI), C105Y (CSIPPEVKFNKPFVYLI), mitoparan (INLKKLAKL(Aib)KKIL), nosangiotide (RKKTFKEVANAVKISA), cytochrome C**⁵⁻¹³** (KGKKIFIMK).

The peptide may comprise or consist of the following amino acid sequence:
RQIKIWFQNRRMKWKKKPNATRPVTPPRVASGLNPSIQKASNYRNNTVLY-

This sychnological tandem construct^{[5,6,9]} combines the CPP Penetratin^{[13]} (RQIKIWFQNRRMKWKK) with a sequence derived from the carboxyl-terminal of PPP1 (KPNATRPVTPPRVASGLNPSIQKASNYRNNTVLY). This peptide, synthesized as a tandem linear construct, was prepared with an amidated carboxyl terminal on a Rink amide solid phase.

The peptide may comprise or consist of the following amino acid sequence:
GQQDQDRKVICFVDVSTLNVRQIKIWFQNRRMKWKK

This sychnological tandem construct ^{[5,6,19]} combines the sequence of the CPP Penetratin^{[13]} (RQIKIWFQNRRMKWKK) in the carboxyl terminal with a sequence derived from AKAP4^{[7]} (GQQDQDRKVICFVDVSTLNV) as an amino-terminal extension. The latter sequence contains the RVxF motif KVICF^{[18]}. This bioportide, synthesized as a tandem linear construct, was prepared with an amidated carboxyl terminal on a Rink amide solid phase.

The peptide may further comprise a cell-penetrating peptide with a PIP binding site motif (sequence derived from the binding site between PPP1 and a PIP).

The PIP binding site motif may be selected from the group consisting of **PPP1CC2 C-terminus,** K-P-N-A-T-R-P-V-T-P-P-R-V-A-S-G-L-N-P-S-I-Q-K-A-S-N-Y-R-N-N-T-V-L-Y; **AKAP4**^{[7]}, G-Q-Q-D-Q-D-R-K-V-I-C-F-V-D-V-S-T-L-N-V, K-V-I-C-F; **RVxF**^{[18]}, [RK]-X(0,1)-[VI]-{P}-[FW], [HKR]-[ACHKMNQRSTV]-V-[CHKNQRST]-[FW], [KRL]-[KRSTAMVHNQ]-[V₆]-{FIMYDP}-[FW]; **SILK**^{[8]}, K-[GS]-I-L-K, [GS]-I-L-[RK]; **PPP1R2 degenerate**^{[20]}, R-[KR]-X-H-Y; **iASPP degenerate**^{[21]}, R-N-Y-F; **MyphoNE**^{[19]}, R-X-X-Q-[VIL]-[KR]-X-[YW]; **Apoptotic signature**^{[22]}, F-X-X-[RK]-X-[RK], **Apoptotic signature with juxtaposed RVxF**^{[23]}, [RK]-X(0,1)-[VI]-X-F-X-X-[RK]-X-[RK]; **GADD34**^{[24]}, R-A-R-A; and **SpiDoc**^{[25]}, Y-S-N-E-D-Y-D-R.

The preferred methodology for the synthesis of PPP1-modulatory bioportides is microwave-enhanced solid phase peptide synthesis^{[26]}. Solid phase synthesis may be commenced from the C-terminal end of the peptide by coupling a protected alpha-amino acid to a suitable resin. Microwave assisted peptide synthesis may be performed using Microwave Peptide Synthesizer, for example a Liberty peptide synthesizer (CEM Corporation, Matthews, NC), employing methods that control a reaction at a set temperature for a set amount of time. Typically, amino acids are coupled onto Fluorenylmethyloxycarbonyl (Fmoc)-protected 4-methylbenzhydrylamine resin with the Rink amide linker to provide peptide amides after cleavage with trifluoroacetic acid (TFA). Coupling reactions utilise the N-α-Fmoc-protected form of amino acid or mimetic, with 5 equivalents of amino acid and a suitable coupling reagent. After coupling, the resin may be washed and dried under vacuum. Loading of the amino acid onto the resin may be quantified by determination of released Fmoc groups by UV analysis. PPP1-modulatory bioportides may be synthesized as a single conventional peptide, where all amino acids are joined by a conventional peptide bond, or by the chemical ligation of peptide fragments^{[27]}.

The disclosure, which does not form part of the invention, relates to bioportides designed to disrupt PPIs between PPP1CC2 and PIPs may be constructed according to a rhegnylogic strategy^{[5,6,19]}. In this case the pharmacophores that enable translocation into sperm and those that recognize and mimic PPI sites are discontinuously organized within a single peptide. An example would be Tat¹⁻⁷SpiDoc⁸⁻¹⁵Tat¹⁶⁻²⁴: GRKKRRQYSNEDYDRRRRPQRNYF. In this sequence the SpiDoc PIP binding site motif^{[25]} (YSNEDYDRR) is flanked by amino acids derived from the Tat CPP^{[12]}.

The disclosure, which does not form part of the invention, relates to bioportides designed to disrupt PPIs between PPP1CC2 and PIPs may be constructed as proteomimetic mimetics of the primary sequence of either PPPI or any PIP. An example is KPNATRPVTPPRVASGL a polycationic sequence within the C-terminal of PPP1CC2.

The disclosure, which does not form part of the invention, relates to bioportides designed to disrupt PPIs between PPP1CC2 and PIPs may be constructed by combining multiple copies of a PIP binding site motifs as a single chimeric sequence. For example, motifs A and B could be combined as AA, AB, BA, BB, AAA, AAB, ABA BAA, ABB, BAB, BBA and BBB etc.

According to another aspect of the invention, there is provided a peptide according to the invention for use in modulating phosphoprotein phosphatase 1 complexes.

According to another aspect of the invention, there is provided a peptide according to the invention for use in modulating sperm motility. Preferably, the peptide according to the invention is for use in inhibiting and/or reducing sperm motility. Preferably, the peptide according to the invention is for use in increasing or decreasing sperm motility.

As used herein, 'sperm motility' refers to the flagellar/swimming motion of the sperm cells.

According to another aspect of the invention, there is provided a peptide according to the invention for use in the treatment of male infertility.

The disclosure, which does not form part of the invention, relates to a peptide according to the invention for use as a male contraceptive.

The disclosure, which does not form part of the invention, relates to a male contraceptive comprising a peptide according to the invention.

According to another aspect of the invention, there is provided a method of providing contraception, comprising administering an effective amount of a peptide according to the invention to a subject in need of male contraception.

As used herein, a 'subject' can be any mammal or animal including, for example, farm animals, such as sheep, pigs, cows and horses; pet animals, such as dogs and cats; laboratory animals, such as rats, mice and rabbits. The mammal may be a human.

The invention may further provide a pharmaceutical composition comprising a peptide according to the invention and a pharmaceutically acceptable carrier. The pharmaceutically acceptable carrier may be, but is not limited to, sucrose, starch, and other such excipients; cellulose, methyl cellulose, and other such binders; starch, carboxymethyl cellulose, and other such disintegrating agents; magnesium stearate, Aerosil, and other such lubricants; citric acid, menthol, and other such flavoring agents; sodium benzoate, sodium bisulfite, and other such preservatives; citric acid, sodium citrate, and other such stabilizers; methyl cellulose, polyvinyl pyrrolidone, and other such suspending agents; surfactants and other such dispersing agents; water, physiological saline, and other such diluents; beeswax, and the like.

The pharmaceutical compositions according to the present invention may be employed in any form suitable for effective modulation (increase/decrease) of sperm motility. For example, the pharmaceutical compositions of the invention could be in various forms known to the art, including capsules, tablets, liquid form or in lotion form, either oil-in water or water-in-oil emulsions, in aqueous gel compositions, in the form of foams, films, sprays, ointments, suppository, jellies, creams, liposomes or in other forms embedded in a matrix for the slow or controlled release of the peptide to the skin or surface onto which it has been applied or in contact, The pharmaceutical compositions of the invention may be ointments, jellies or creams.

The pharmaceutical compositions according to the present invention can be prepared with carriers that protect them against rapid elimination from the body, such as coatings or time-release formulations. Such carriers include controlled release formulations, such as, but not limited to, microencapsulated delivery systems.

A pharmaceutical composition according to the present invention may be intended to be administered orally, parenterally, intravenously, intradermally, subcutaneously, transdermally, intranasally or topically, in liquid or solid form. Preferably, the pharmaceutical composition according to the present invention is administered topically.

The peptides may be of the invention are included in the pharmaceutically acceptable carrier in an amount sufficient to deliver to a subject a therapeutically effective amount of the peptide to increase or decrease sperm motility without causing any serious toxic effects in the treated subject.

The peptide according to the invention can be used in a coating composition within a condom.

It will be appreciated that optional features applicable to one aspect or embodiment of the invention can be used in any combination, and in any number. Moreover, they can also be used with any of the other aspects or embodiments of the invention in any combination and in any number. This includes, but is not limited to, the dependent claims from any claim being used as dependent claims for any other claims of this application.

Embodiments of the present invention will now be described, purely by way of example, with reference to the accompanying drawings, in which:

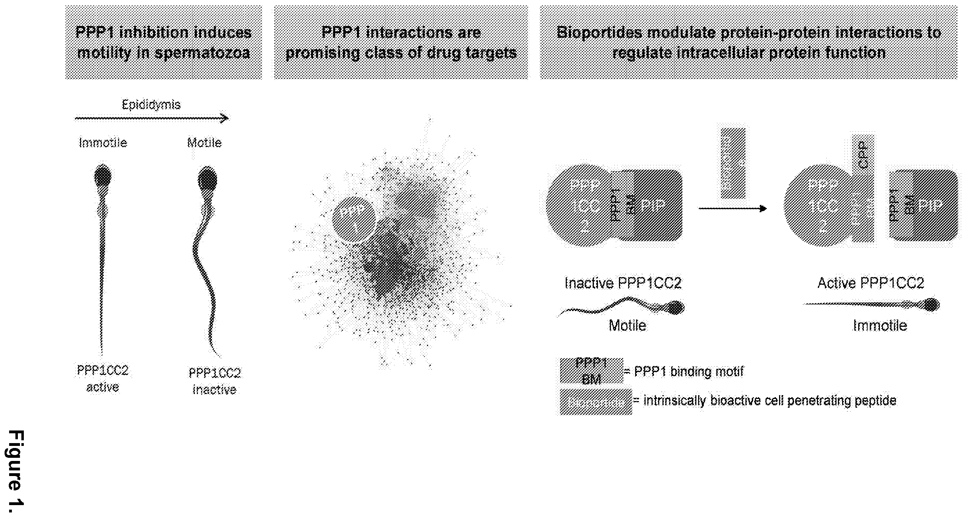

**Figure 1** - shows the acquisition of sperm motility within the epididymis is regulated by the activity of a sperm-specific subtype of PPP1 (PPP1 catalytics subunit gamma 2 (PPP1CC2)). The function of the PPP1CC2 protein is controlled by a variety of interacting proteins (PIPs).

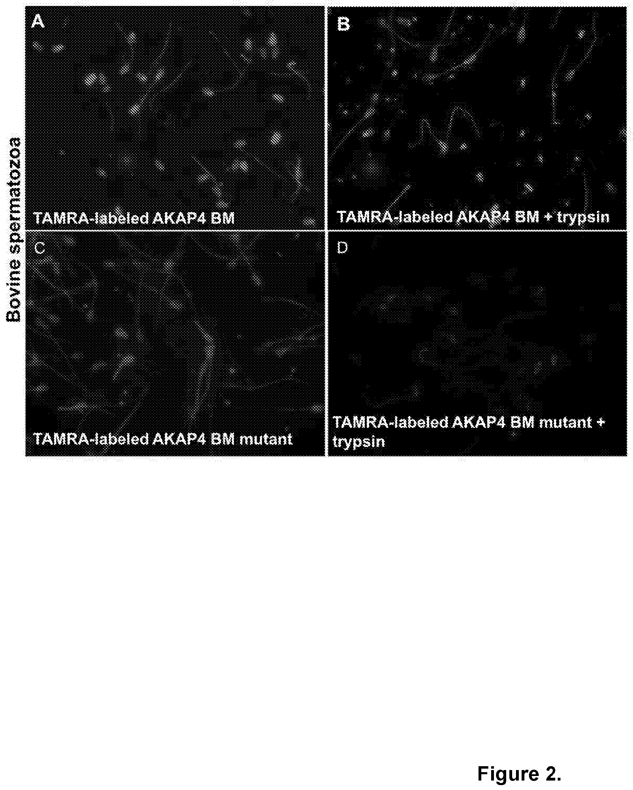

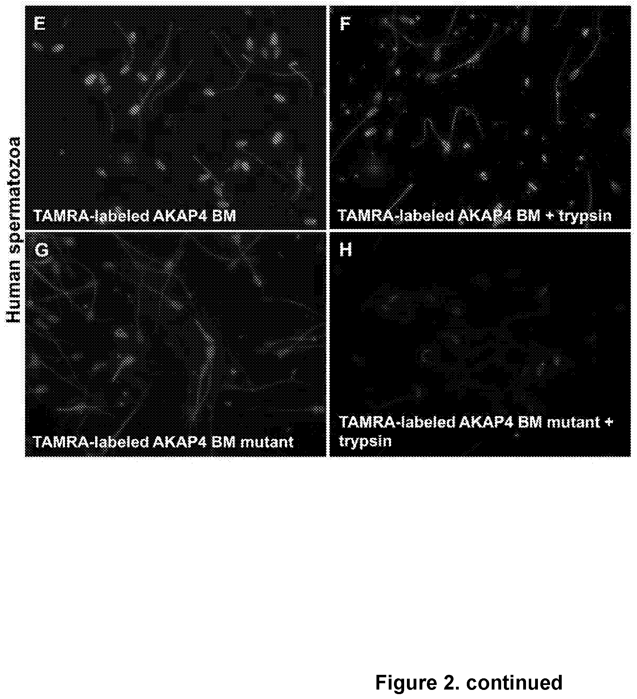

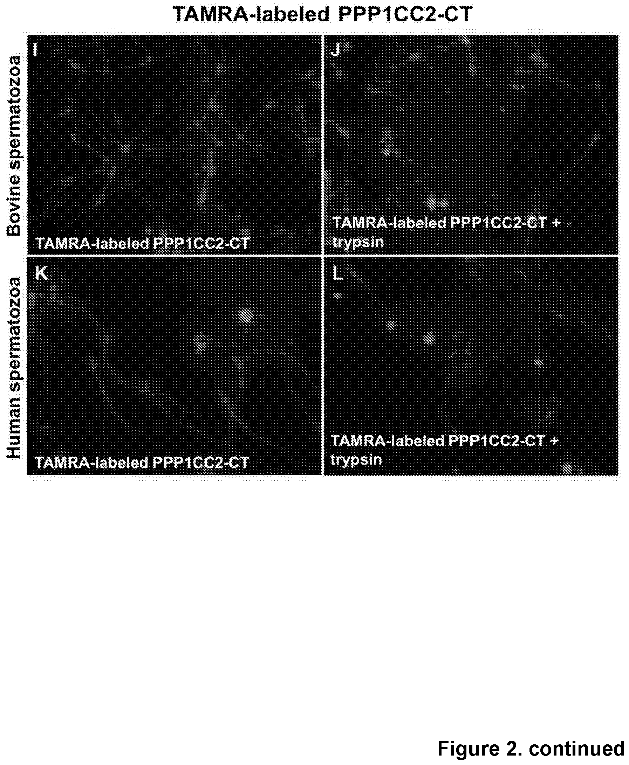

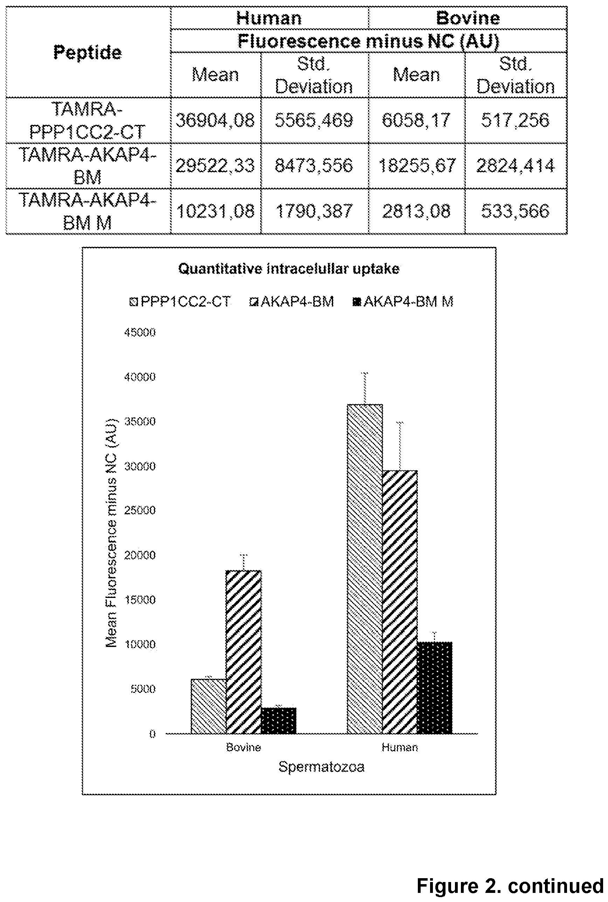

**Figure 2** - shows the intracellular accumulation of the TAMRA-labelled bioportides within bovine and human spermatozoa. To establish that the detected fluorescence was not attributed to surface-associated peptide, isolated sperm cells were incubated for 1h with 10µM TAMRA-labelled peptide and subsequently treated with 1% (w/v) trypsin to remove any peptide bound to the surface of spermatozoa. Negative controls without the TAMRA-labelled peptides were performed and presented no reactivity. Images are representative from three independent experiments. Lower panel (right): Quantitative analyses of peptide translocation into bovine and human spermatozoa. Bovine and human spermatozoa were incubated with TAMRA-labelled bioportides (10µM) for 1 h at 37°C. Data are expressed as fluorescence (minus negative control). Three independent experiments were performed in quadruplicate. Error bars 95% CI.

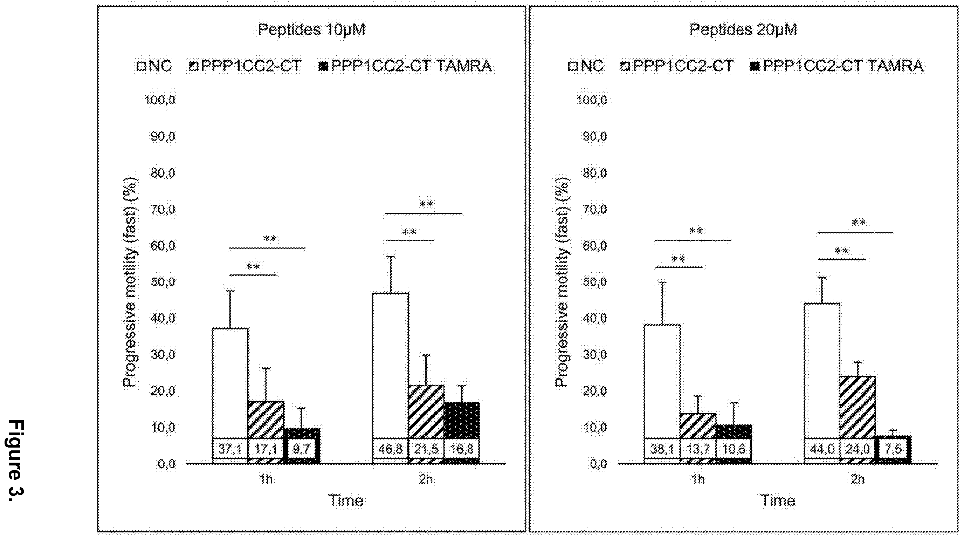

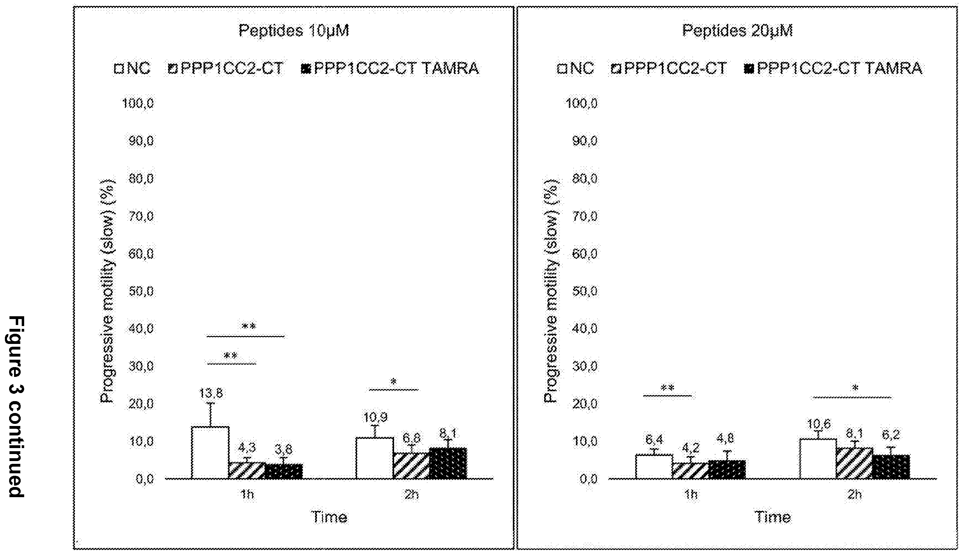

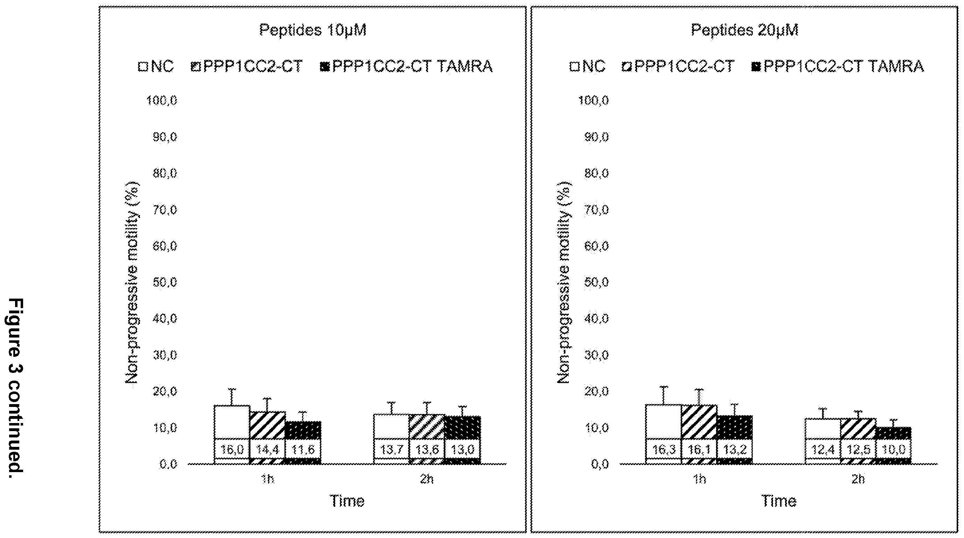

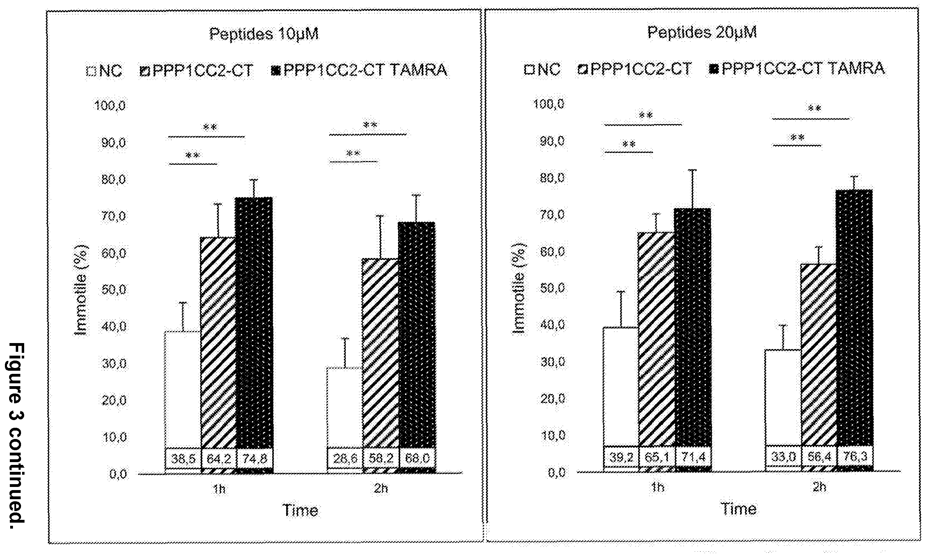

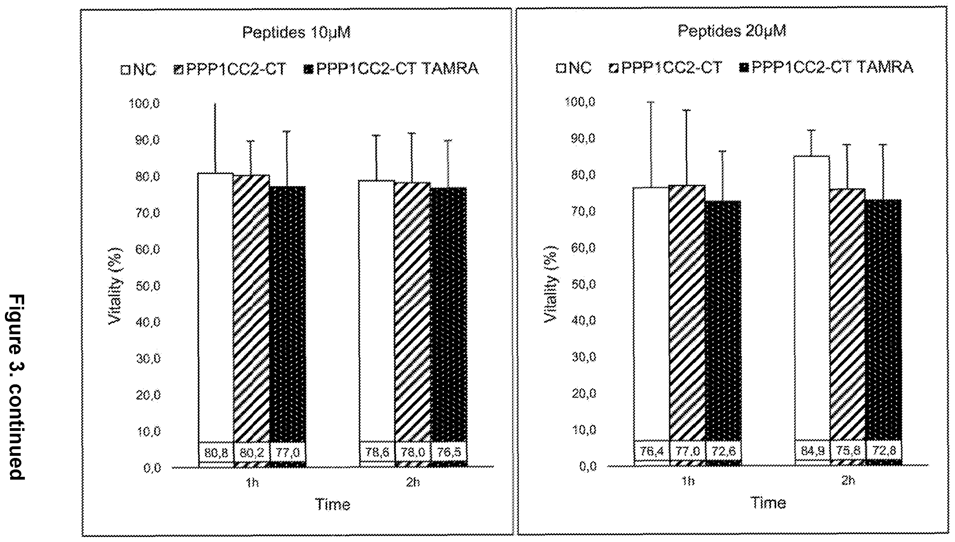

**Figure 3** - shows the impact of the PPP1CC2-CT peptides (PPP1CC2-CT and TAMRA-PPP1CC2-CT) treatment in bovine spermatozoa motility and viability parameters: (A) fast progressive motility; (B) slow progressive motility; (C) non-progressive motility; (D) immotile spermatozoa; and (E) viability. Graph bars represent the mean values of three independent experiments performed in triplicate. Error bars 95% CI. Statistically significant findings are indicated with a (*). *P<0.05; **P<0.01.

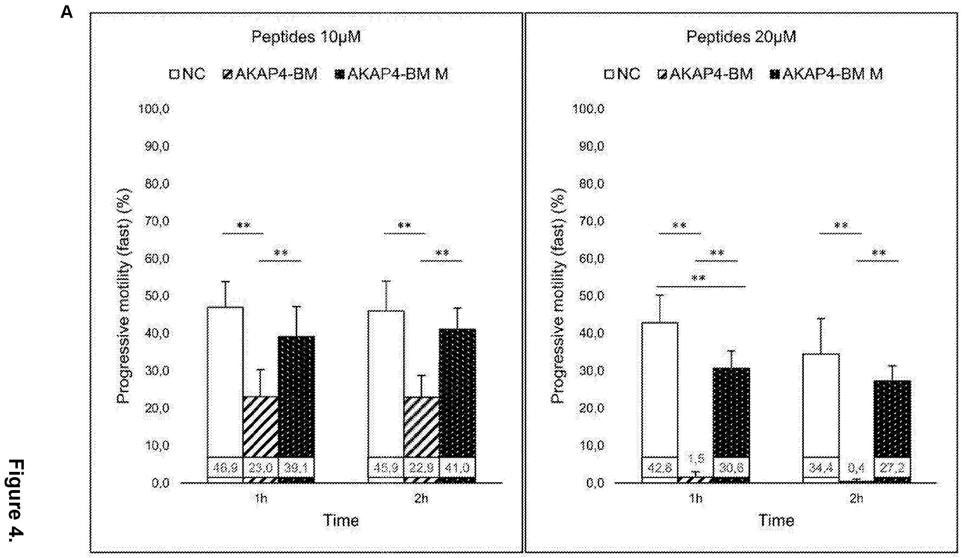

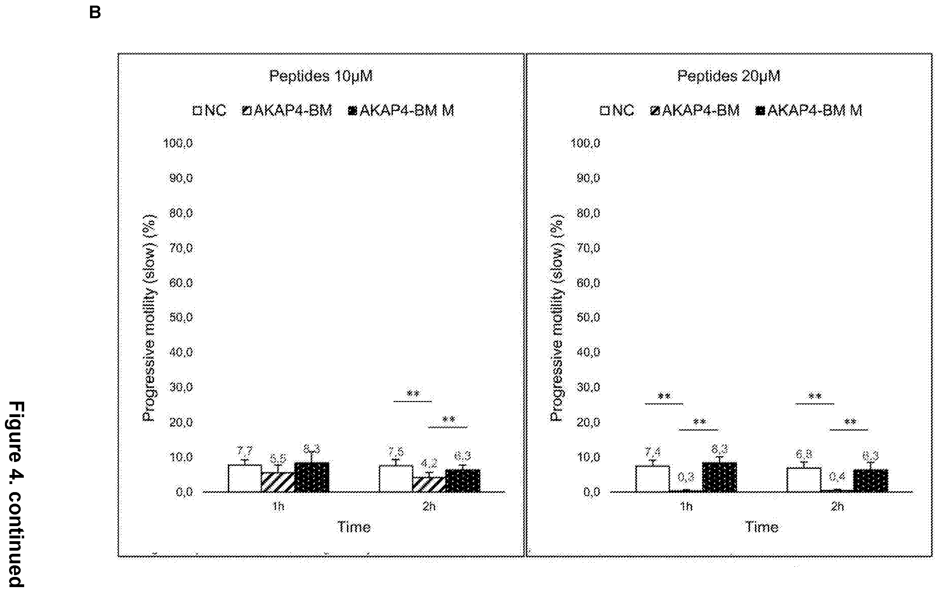

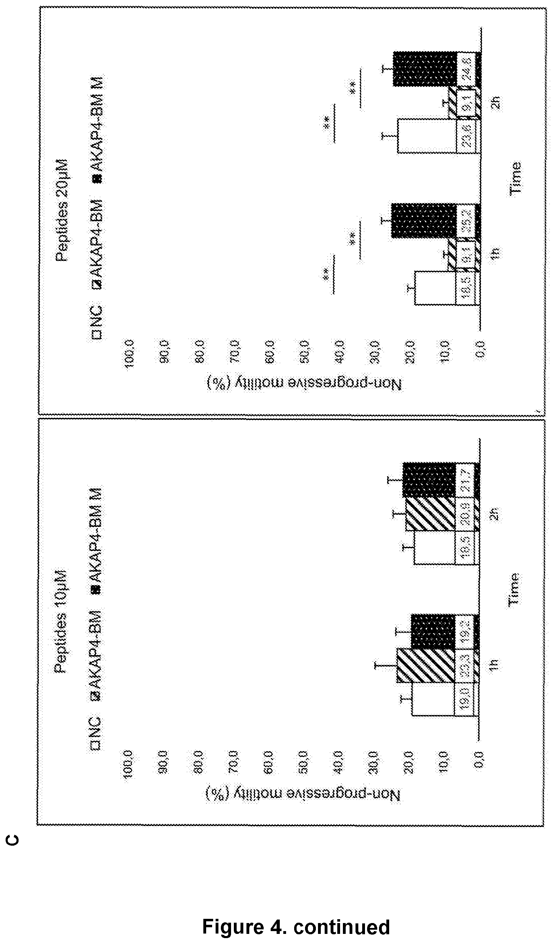

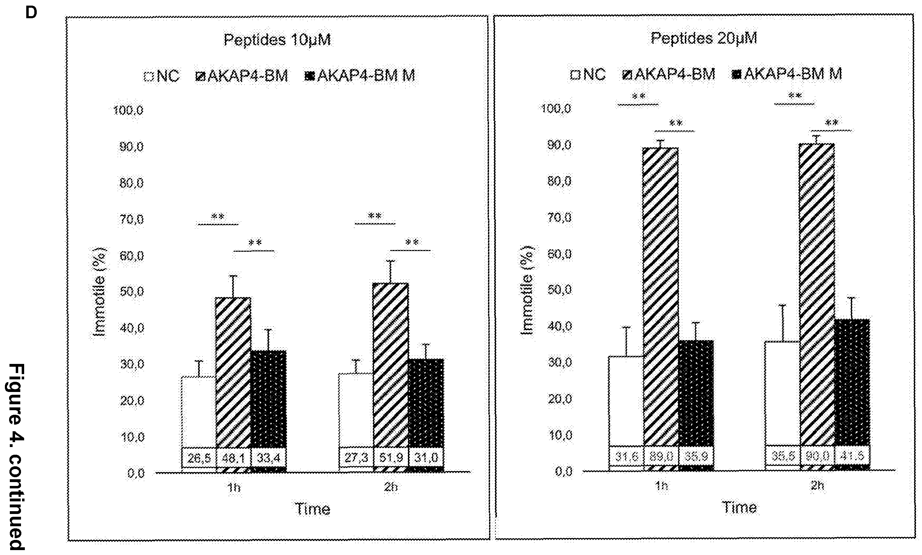

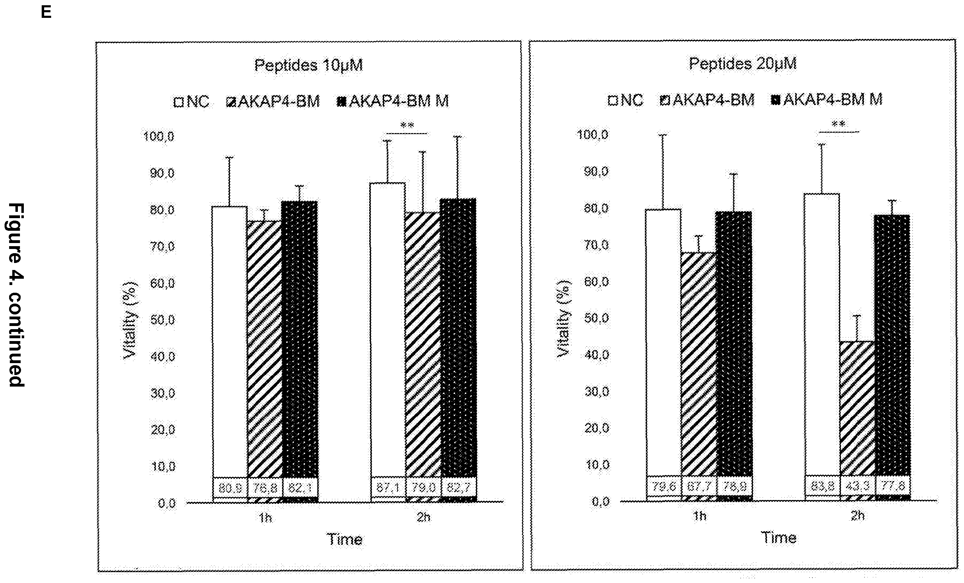

**Figure 4** - shows the impact of the AKAP4-BM peptides (AKAP4-BM and AKAP4-BM M) treatment in human spermatozoa motility parameters: (A) fast progressive motility; (B) slow progressive motility; (C) non-progressive motility; (D) immotile spermatozoa; and (E) viability. Graph bars represent the mean values of three independent experiments performed in triplicate. Error bars 95% CI. Statistically significant findings are indicated with a (*). *P<0.05; **P<0.01.

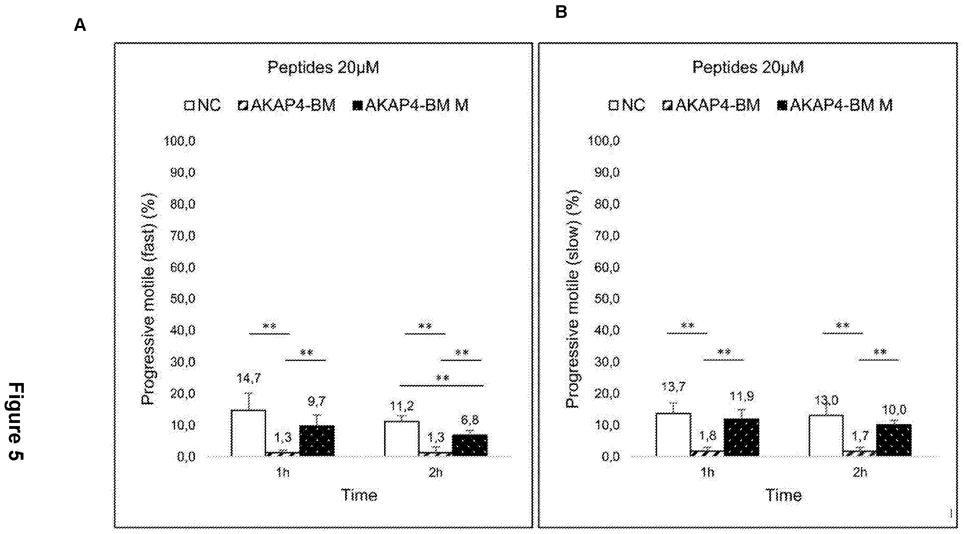

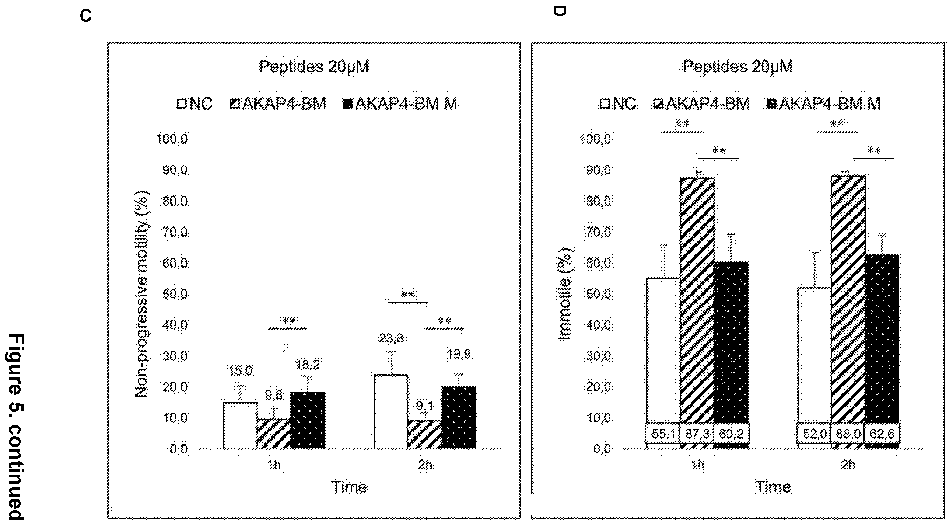

**Figure 5** - shows the impact of the AKAP4-BM peptides (AKAP4-BM and AKAP4-BM M) treatment in human spermatozoa motility parameters: (A) fast progressive motility; (B) slow progressive motility; (C) non-progressive motility; (D) immotile spermatozoa; and (E) viability. Graph bars represent the mean values of three independent experiments performed in triplicate. Error bars 95% CI. Statistically significant findings are indicated with a (*). *P<0.05; **P<0.01.

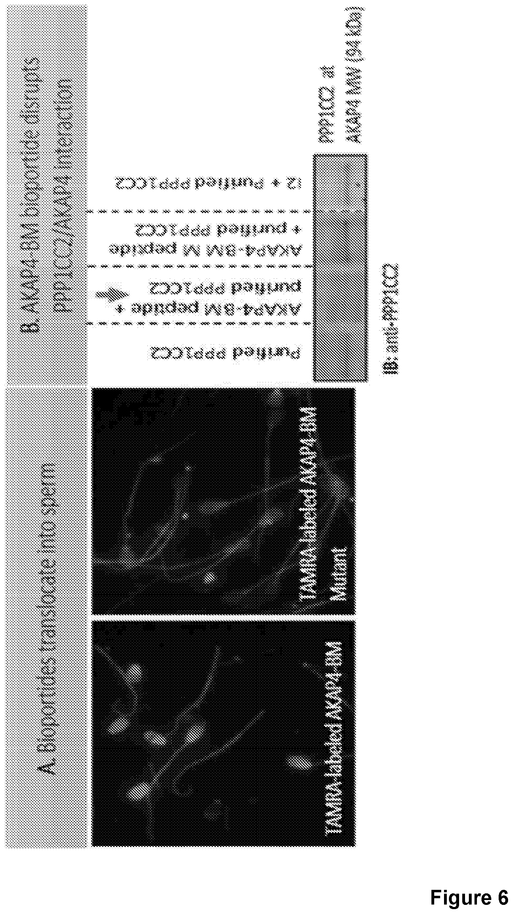

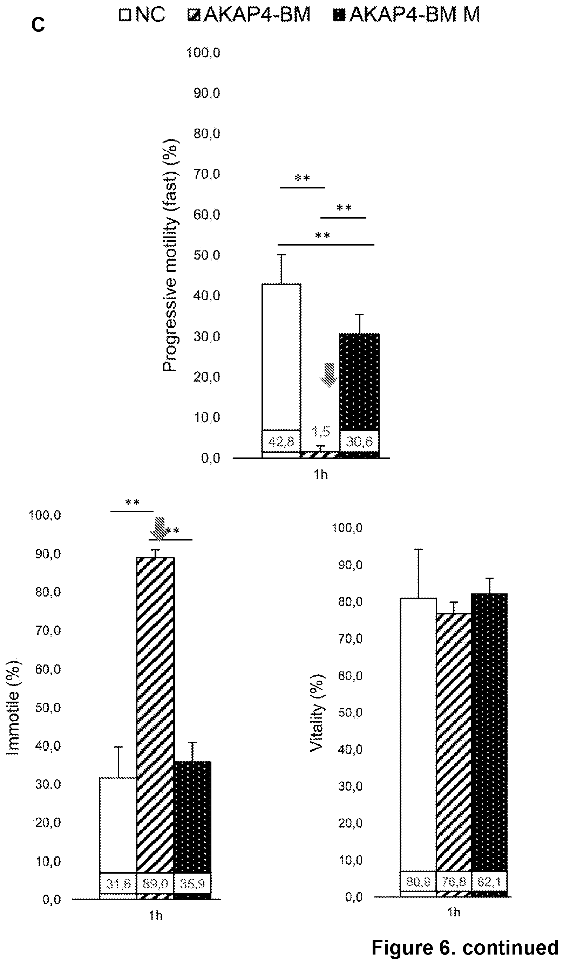

**Figures 6A****-C** - Figure 6A shows red fluorescent (TAMRA) analogues of AKAP4-BM and AKAP4 BM M peptides (5 µM) accumulate within normal healthy human spermatozoa. Intracellular distribution of TAMRA-labelled bioportides in human sperm is comparable to results obtained with bovine sperm. Images are representative from 3 independent experiments. Figure 6B shows that the AKAP4-BM bioportide was able to disrupt PPP1CC2-AKAP4 complex (in the presence of the peptide - lane 2 - the interaction between PPP1CC2 and AKAP4 is reduced). A PPP1CC2-AKAP4 complex was purified from human sperm (Lane 1, left). Co-incubation of this protein complex with the AKAP4-BM bioportide (10 µM) disrupts the PPP1CC2-AKAP4 complex (Lane 2), A peptide with a mutation in the PPP1 binding motif of AKAP4 (AKAP4-BM M) was used as a control (lane 3). 12, a PPP1 inhibitor, was used as a positive control and, as expected, also decreases the interaction (lane 4). Figure 6C shows that exposure of sperm to AKAP4-BM bioportide induced a decrease in progressive motility and an increase in immotile spermatozoa. Application of the mutated AKAP4-BM did not lead to significant differences. The percentage of viable spermatozoa was not significantly changed after treatment with peptides. Graph bars represent 3 independent experiments performed in triplicate. **p<0.01.

The invention now being generally described, it will be more readily understood by reference to the following examples which are included merely for purpose of illustration of certain aspects and embodiments of the present invention, and are not intended to limit the invention in any way.

### Materials and Methods

### Microwave-assisted solid phase peptide synthesis

Peptide sequences derived from (a) the 22 amino acid C-terminus of the human PPP1CC2 (³⁰³KPNATRPVTPPRVASGLNPSIQKASNYRNNTVLY³³⁶) [PPP1CC2-CT], (b) the region including the PPP1 binding motif (⁴⁰KVICF⁴⁴) of the human AKAP4 (³³GQQDQDRKVICFVDVSTLNV⁵²) [AKAP4-BM] and (c) a mutated homologue (³³GQQDQDRAAAAAVDVSTLNV⁵²) [AKAP4-BM M] were synthesized using microwave-assisted solid-phase peptide synthesis. These endogenous sequences were coupled to the Penetratin CPP (RQIKIWFQNRRMKWKK; Table 1) as sychnological tandem constructs.

As recently described^{[3,26,29]}, microwave-assisted solid phase peptide synthesis was performed using a Discover SPS Microwave Peptide Synthesizer (CEM Microwave Technology Ltd, Buckingham, UK) with fibre optic temperature control. Peptides were synthesized (0.1 mmol scale) using Rink amide MBHA resins pre-loaded with the first amino acid (AnaSpec, Inc., Cambridge Bioscience Ltd, Cambridge, UK) and employed an N-α-Fmoc protection strategy with HCTU activation. Deprotection with 7 ml of 20 % piperidine was performed for 3 min at 50 W/75 °C A majority of AA coupling reactions were accomplished with a 4-fold molar excess of Fmoc-protected AA with HCTU and diisopropylethylamine (DIPEA), molar ratio of 1:1:2 (AA/HCTU/DIPEA), in 4 ml for 10 min at 25 W/75 °C. Arg coupling was performed in two stages: 30 min 0 W/∼25 °C followed by 5 min at 17 W/75 °C. To reduce racemization of Cys and His, coupling conditions were 5 min at 0 W/∼25 °C followed by 6 min at 17 W/50 °C with the hindered base collidine (TMP) at a molar ratio of 1:1:2 (AA/HCTU/TMP; ²⁹). Aspartimide formation was reduced by the substitution of piperidine for 5 % piperazine and 0.1 M 1-hydroxybenztriazole hydrate (HOBt) in the deprotection solution.

Fluorescent peptides, to be used in cell imaging and quantitative uptake analyses, were synthesized by amino-terminal acylation with 6-carboxytetramethylrhodamine (6-carboxyTAMRA) (Novabiochem, Beeston, UK) as previously described^{[26,29]}. Peptides were purified by semi-preparative scale high-performance liquid chromatography, and the predicted masses of all peptides were confirmed by matrix-assisted laser desorption ionization (MALDI) time of flight mass spectrometry. Sequences, abbreviations and masses of all peptides used in this study are shown in Table 1.

**Table 1. Peptide sequences, abbreviations and molecular masses. To enable both quantitative and qualitative uptake analysis, peptides were extended with TAMRA. Peptide masses (M+H⁺) were confirmed by MALDI time of flight mass spectrometry. Underlined, penetratin (CPP); Bold, PPP1 binding motif (pattern: [RK]-X(0,1)-[VI]-{P}-[FW]). The final amino acid of the native PPP1CC2 carboxyl-terminus (glutamic acid) was excluded due to its negative charge. All peptides were synthesized with a free-amine as the amino terminal (-H) and an amidated carboxyl-terminal (-NH₂).**

| **Peptide designation** | **Abbreviation** | **Peptide sequence** | **Mass (g/mol)** |
|---|---|---|---|
| PPP1CC2 C-terminal | PPP1CC2-CT | H-RQIKIWFQNRRMKWKKKPNATRPVTPPRVAS GLNPSIQKASNYRNNTVLY-NH2 | 5953.0 |
| AKAP4 binding motif | AKAP4-BM | H-GQQDQDRKVICFVDVSTLNVRQIKIWFQNRR MKWKK-NH2 | 4448.3 |
| AKAP4 binding motif mutant | AKAP4-BM M | H-GQQDQDRAAAAAVDVSTLNVRQIKIWFQNRR MKWKK-NH2 | 4212.9 |

### Semen samples and preparation

This study was approved by the Ethics and Internal Review Board of the Hospital Infante D. Pedro E.P.E. (Aveiro, Portugal) and was conducted in accordance with the ethical standards of the Helsinki Declaration. Human semen samples were obtained from a randomized group of donors, by masturbation to a sterile container. All donors signed informed consent allowing the samples to be used for scientific purposes. Basic semen analysis was performed according to World Health Organization (WHO) guidelines. Fresh semen from Holstein Frisian bulls was obtained from LusoGenes, LDA (Aveiro, Portugal). Semen was collected by artificial vagina and assessed by a certified veterinarian. Human and bovine spermatozoa were isolated and washed from seminal plasma by centrifugation (800 g for 5 min, 3 times) using ALLGrade Wash medium (LifeGlobal, Brussels, Belgium). Spermatozoa pellets were resuspended in medium to a final concentration of 20x10⁶ cells/0.5 mL and incubated at 37°C with 5 % CO₂ until the appropriated treatments were added.

### Bioportides translocate into spermatozoa

It has been recently demonstrated that cell-penetrating peptides accumulate within discrete intracellular compartments of bovine spermatozoa^{[3]} and that proteomimetic bioportides are able to regulate Ca²⁺-signalling events in human sperm^{[28]}. In order to employ bioportides in human sperm a requisite first step is to determine whether they can penetrate the unique plasma membrane that surround a human sperm and accumulate within intracellular sites coincident with target protein. Confocal microscopy^{[13]} is the method of choice for these studies and can be employed with both living and fixed spermatozoa. CPPS and bioportides that are labelled with tetramethylrhodamine^{[13,29]} (TAMRA) at the amino-terminal were utilised to take advantages of the spectral properties of a red-fluorescent chromophore.

### Confocal analyses of the intracellular accumulation of the peptides

Isolated bovine and human spermatozoa (4 x10⁷ cells) were suspended and incubated with 10 µM TAMRA-labeled peptides for 1 h at 37 °C in a humidified atmosphere of 95% air/5 % CO₂. Cells were then washed three times (800 g for 5 min) in ALLGrade Wash medium (LigeGlobal, Brussels, Belgium). To confirm if the fluorescently labeled peptides were not merely surface associated, the sperm cell population was divided in two and a trypsin incubation was used to remove any surface associated peptides. In summary, half of the cells were set aside (2 x10⁷ cells) and fixed in 4 % paraformaldehyde (PFA) for 20 min. The other half was incubated with 1 % (wt/vol) trypsin at 37 °C, collected by centrifugation at 3000 g, washed in ALLGrade Wash medium (LigeGlobal, Brussels, Belgium) and resuspended in 4 % PFA 20 min. Fixed cells from each preparation were spread into coverslips and allowed to air dry. After mounting, slides were assessed by fluorescence microscopy (Imager.Z1, Axio-Cam HRm camera and AxioVision software, Zeiss, Jena, Germany).

### Quantitative analyses of peptide translocation

Translocation efficacies of CPP-mediated intracellular delivery of the peptides sequences were determined by the quantitative uptake analysis of fluorescently labeled moieties^{[3]}. Isolated bovine and human spermatozoa were incubated with 10 µM TAMRA-labeled peptides for 1 h at 37 °C in a humidified atmosphere of 5 % CO₂. Cells were then washed four times with PBS, detached with 1 % (w/v) trypsin at 37 °C, collected by centrifugation at 3000 g and lysed in 300 µl 0.1 M NaOH for 2 h on ice. 250 µl of each sample cell lysate were transferred to a black 96-well plate, and analyzed using an Infinite® 200 PRO (Tecan, Switzerland) (λAbs 544 nm/λEm 590 nm).

### Cell viability assays

Bovine spermatozoa viability was evaluated using the trypan blue viability test according to manufacturer's guidelines. Human spermatozoa viability was measured using the CellTiter 96® AQueous Non-Radioactive Cell Proliferation Assay (Promega, Madison, USA) according to manufacturer's guidelines.

### Motility assays

Peptides (PPP1CC2-CT, TAMRA-PPP1CC2-CT, AKAP4 and AKAP4 M) were added to bovine and human sperm samples in total volume of 500 µl containing approximately 2 x10⁷ sperm cells per well. Control samples included sperm cells in medium alone. The influence of bioportides on sperm motility parameters was assessed using the Sperm Class Analyzer CASA System (Microptic S L, Barcelona, Spain) with SCA® v5.4 software. Samples and controls (2 µl) were loaded into individual chambers of Leja Standrat Count 8 chamber slide 20 µm depth (Leja Products B. V., The Netherlands) which were pre-heated at 37 °C. This temperature was maintained while at least 1000 sperm cells/per measurement were evaluated. Each peptide was tested on samples from 3 individual donors and all the conditions were performed in triplicate.

### Results

Cell penetrating peptides were used to deliver peptide sequences according to the invention into sperm cells with the aim of modulating their motility. A peptide sequence that mimics the unique 22 amino acid C-terminus of PPP1CC2 (PPP1CC2-CT), conserved in both bovine and human, was used to compromise the isoform-specific interactions between PPP1CC2 and its interactors. The AKAP4-BM peptide mimics the PPP1 binding motif RVxF in AKAP4 (⁴⁰KVICF⁴⁴), which is also conserved in both species, and was used to disrupt PPP1CC2-AKAP4 complexes. A peptide with a mutation in the PPP1 binding motif of AKAP4 (AKAP4-BM M) was used as a control of the AKAP4-BM. A negative control (samples without any treatment) was used in all the experiments.

### Peptides translocate into bovine and human spermatozoa

Fluorescent microscopy clearly revealed the intracellular accumulation of the peptides within bovine and human spermatozoa (Figure 2). Quantitative uptake analyses also demonstrated a range of translocation efficacies amongst those peptides tested (Figure 2). To confirm that the fluorescently labeled peptides were not merely surface associated, peptide-treated spermatozoa were firstly incubated with high concentrations of exogenous trypsin that rapidly degrades polycationic peptides as previously described^{[3]}. Microscopic analyses showed that the PPP1CC2-CT and AKAP4 BM peptides internalized very efficiently in both human and bovine spermatozoa, while the AKAP4 BM M peptide internalized less efficiently giving rise to a reduced fluorescent signal (Figure 2). Quantitative comparison of the uptake efficacies (Figure 2) was in accordance with the microscopic observations. The PPP1CC2-CT and AKAP4 BM peptides revealed higher cellular uptake, when compared to the AKAP4 BM M peptide, probably reflecting specific binding of PPP1CC2-CT and AKAP4 BM peptides after membrane translocation. The uptake of the peptides differed between species, with human spermatozoa revealing higher uptake of all the peptides when compared with bovine cells.

### Bioportides influence sperm motility

Sperm class analysis data yields useful information about the fertility status of an individual and is often employed in the primary diagnosis of male factor infertility^{[30]}. To confirm that bioportide abrogate the motility of bovine and human spermatozoa, samples were treated with bioportides and motility characterised by a sperm class analyser.

Sperm motility, a prognostic parameter of human fertilization capacity and a major determinant of assisted conception, is regulated by the activity of the sperm-specific subtype of phosphoprotein phosphatase 1 (PPP1) isoform PPP1CC2. The activity of this phosphatase, differentially expressed in spermatozoa, is regulated by a number of PPP1CC2 Interacting Proteins (PIPs). Changes in the activity of PPP1CC2, regulated by interactions with PIPs, control the acquisition of sperm motility prior to ejaculation. Hence, the development and application of bioportides, which act by a dominant negative mode of action upon sperm-specific PPIs between PPP1CC2 and various PIPs, is a unique approach to the regulation of sperm motility. Some cationic sequences, often corresponding to domains involved in PPIs, are intrinsically cell permeable. Alternatively, impermeable sequences can be delivered into human spermatozoa conjugated to an inert CPP vector.

### Impact of the peptides on sperm motility

Bovine and human spermatozoa were exposed to different extracellular peptide concentrations (10 and 20 µM) and motility parameters were assessed at 1 and 2 h. For reference, negative controls were performed in the absence of peptides.

The percentage of viable spermatozoa was not significantly changed after treatment with peptides, except by the 2h incubation with the AKAP4-BM peptide where a slight decrease was observed (Figure 3).

### PPP1CC2-CT peptide

Exposure of bovine spermatozoa to 10 µM PPP1CC2-CT induced a decrease in both fast (mean decrease at 1h of 55.9 ± 19.4%; at 2h 53.9 ± 19.9%; compared with negative control) and slow (mean decrease at 1h of 49.9 ± 41%; at 2h 17.4 ± 57%; compared with negative control) progressive motility. Incubation of bovine spermatozoa with 20 µM PPP1CC2-CT induced similar effects: decrease in both fast (mean decrease at 1h of 64.2 ± 8.3%; at 2h 44.6 ± 9.7%; compared with negative control) and slow (mean decrease at 1h of 31.4 ± 35%; compared with negative control) progressive motility. In contrast, immotile spermatozoa increased with both 10 µM (mean increase at 1h of 72.5 ± 33.6%; at 2h 128.3 ± 85.3%; compared with negative control) and 20 µM PPP1CC2-CT (mean increase at 1h of 86.2 ± 72.3%; at 2h 79.1 ± 37,0%; compared with negative control). No significant alterations were observed in non-progressive motility (Figure 3).

Both PPP1CC2-CT and TAMRA-PPP1CC2-CT induced a similar effect on sperm motility parameters (Figure 3), revealing that the fluorescent labelling did not affect significantly peptides action.

There were no significant differences between the concentrations tested (10 and 20 µM) for any motility parameter, suggesting that there was no concentration-dependent effect of the PPP1CC2-CT peptides for the concentrations tested.

Exposure of human spermatozoa to 10 µM PPP1CC2-CT did not induce any significant alterations in the motility parameters. Exposure of sperm to 20 µM PPP1CC2-CT induced a decrease in fast progressive motility at 2h (mean decrease of 34.5 ± 12.3%; compared with negative control).

### AKAP4-BM peptide

Exposure of bovine spermatozoa to 10 µM AKAP4-BM induced a decrease in both fast (mean decrease at 1h of 41.9 ± 20.0%; at 2h 43.9 ± 15.3%; compared with AKAP4-BM M; mean decrease at 1h of 49.8 ± 22.5%; at 2h 50.5 ± 9.7%; compared with negative control) and slow (mean decrease at 2h 21.6 ± 64.4%; compared with AKAP4-BM M; mean decrease at 2h 42.4 ± 20.4%; compared with negative control) progressive motility. Incubation with 20 µM AKAP4-BM induced a decrease in both fast (mean decrease at 1h of 95.0 ± 6.5%; at 2h 98,4 ± 2,6%; compared with AKAP4-BM M; mean decrease at 1h of 95,7 ± 5.4%; at 2h 98.6 ± 2.9%; compared with negative control) and slow (mean decrease at 1h of 95,7 ± 4.7%; at 2h 94.1 ± 4.9%; compared with AKAP4-BM M; mean decrease at 1h of 95.3 ± 4.8%; at 2h 94.2 ± 4.8%; compared with negative control) progressive motility. In addition, exposure to 20 µM AKAP4-BM peptide led to a significant decrease in non-progressive motility (mean decrease at 1h of 63.1 ± 8.3%; at 2h 63.0 ± 7.5%; compared with AKAP4-BM M; mean decrease at 1h of 50.0 ± 10.9%; at 2h 59.4 ± 13,2%; compared with negative control). In contrast, immotile spermatozoa increased with both 10 µM (mean decrease at 1h of 4.,9 ± 33,2%; at 2h 73.5 ± 43,3%; compared with AKAP4-BM M; mean decrease at 1h of 90.0 ± 51,4%; at 2h 91.8 ± 25,4%; compared with negative control) and 20 µM AKAP4-BM (mean decrease at 1h of 155.7 ± 46,1%; at 2h 123.2 ± 38,1%; compared with AKAP4-BM M; mean decrease at 1h of 91.8 ± 25.4%; at 2h 183.7 ± 88.4%; compared with negative control; Figure 5). Application of the mutated AKAP4-BM did not lead to significant differences in motility parameters in bovine spermatozoa (compared with negative control), with two exceptions (fast progressive motility at 1h with 20 µM incubation and the percentage of immotile spermatozoa with 10 µM incubation at 1h; Figure 4).

Upon exposure to the AKAP4-BM peptide, at both 1h and 2h, the progressive (fast and slow) and the non-progressive motility were higher at a lower peptide concentration (10 µM) compared with the higher concentration (20 µM), while the percentage of immotile spermatozoa was higher with 20 µM peptide incubation, suggesting a concentration-dependent effect for the concentrations tested.

Exposure of human spermatozoa to 20 µM AKAP4-BM induced significant alterations in the motility parameters (Figure 5). A decrease in both fast (mean decrease at 1h of 87.0 ± 4.3%; at 2h 82.7 ± 20.7%; compared with AKAP4-BM M; mean decrease at 1h of 49.8 ± 22.5%; at 2h 50.5 ± 9.7%; compared with negative control) and slow (mean decrease at 1h 85.1 ± 5.2%; at 2h 82.3 ± 11.5%; compared with AKAP4-BM M; mean decrease at 1h 87,6 ± 5,2%; at 2h 85,7 ± 8,7%; compared with negative control) progressive motility was observed. In addition, a significant decrease in non-progressive motility (mean decrease at 1h of 43.4 ± 24.8%; at 2h 50.6 ± 21.0%; compared with AKAP4-BM M; mean decrease at 1h 31.0 ± 26.9%; at 2h 58.6 ± 16.1%; compared with negative control) was also observed. In contrast, immotile spermatozoa increased (mean decrease at 1h of 47.3 ± 19.2%; at 2h 41.9 ± 14.7%; compared with AKAP4-BM M; mean decrease at 1h of 63.0 ± 27.8%; at 2h 74.5 ± 28.3%; compared with negative control). Application of the mutated AKAP4-BM did not lead to significant differences in motility parameters in human spermatozoa (compared with negative control), with one exception (fast progressive motility at 2h with 20 µM incubation).

As quantitatively analysed in Figure 5, exposure of sperm to the AKAP4-BM bioportide but not the AKAP4 BK M control peptide, induced a statistically significant decrease in progressive motility (left panel of Figure 6C) and a corresponding increase in the percentage of immotile spermatozoa (middle panel of Figure 6C). The percentage of viable spermatozoa was not significantly changed after treatment with peptides (right panel of Figure 6C).

### Discussion

The identification and application of intrinsically bioactive CPPs, designated herein as bioportides, is further endorsement of the tremendous clinical potential of CPP technologies. The refinement of proteomimetic bioportides, including sequences that mimic domains involved in protein-protein interactions (PPIs), provides tremendous opportunities to modulate this emergent drug modality in a clinical setting.

Peptides designed to modulate PPIs between PPP1CC2 and PIPs, including AKAP4, are clearly able to influence sperm motility without detrimental influence upon cellular viability. Hence, the potential of peptides to penetrate the plasma membrane surrounding sperm and subsequently influence the activity of the sperm-specific protein target PPPICC2 provides a unique approach to the modulation of sperm motility and male fertility.

### References

[1] Kumar N. & Singh A.K. (2015) Trends of male factor infertility, an important cause of infertility: A review of the literature. J. Hum. Reprod. Sci. 8, 191-6.
[2] Elzanaty S. et al. The impact of epididymal and accessory sex gland function on sperm motility. Hum. Reprod. (2002) 17, 2904-11.
[3] Jones, S. et al. (2013) Intracellular translocation and differential accumulation of cell penetrating peptides in bovine spermatozoa: Evaluation of efficient delivery vectors that do not compromise human sperm motility. Hum. Reprod. 28, 1874-89.
[4] Bechara C, & Sagan S. (2013) Cell-penetrating peptides: 20 years later, where do we stand? FEBSLett. 587, 1693-702
[5] Lukanowska M., Howl J. & Jones, S. (2013) Bioportides: Bioactive cell penetrating peptides that modulate cellular dynamics. Biotechnol. J. 8, 918-930.
[6] Howl J. & Jones S. (2015) Insights into the molecular mechanisms of action of bioportides: a strategy to target protein-protein-interactions. Expert Rev. Mol. Med. Vol 17, e1.
[7] Fardilha M. et al. (2001) Protein phosphatase 1 complexes modulate sperm motility and present novel targets for male infertility. Mol .Hum. Reprod. 17, 466-77.
[8] Silva J.V. et al. (2017) Construction and analysis of a human testis/sperm-enriched interaction network: Unraveling the PPP1CC2 interactome. Biochim. et Biophys. Acta 1861, 375-85.
[9] Howl J. et al. (2012) Bioportide: An emergent concept of bioactive cell penetrating peptide. Cell. Mol. Life Sci. 69, 2951-66.
[10] Jones S. & Howl J. (2011) Enantiomer-specific bioactivities of peptidomimetic analogues of mastoparan and mitoparan: Characterisation of inverso mastoparan as a highly efficient cell penetrating peptide. Bioconjugate Chem. 23, 47-56.
[11] Skene-Arnold, T.D. et al. (2013) Molecular mechanisms underlying the interaction of protein phosphatase-lc with ASPP proteins. Biochem. J. 449, 649-59.
[12] Vivès E., Brodin P. & Lebleu B. (1997) A truncated HIV-1 Tat protein basic domain rapidly translocates through the plasma membrane and accumulates in the cell nucleus. J. Biol. Chem. 272, 16010-7.
[13] Derossi D., Chassaing G. & Prochiantz A. (1998) Trojan peptides: the penetratin system for intracellular delivery. Trends Cell Biol. 8, 84-7.
[14] Rhee M. & Davis P. (2006) Mechanism of uptake of C105Y, a novel cell-penetrating peptide. J. Biol. Chem. 281, 1233-40.
[15] Jones S. et al. (2008) Mitoparan and target-selective chimeric analogues: membrane translocation and intracellular redistribution induces mitochondrial apoptosis. Biochim. Biophys. Acta. Mol. Cell. Res. 1783, 849-63.
[16] Soomets U. et al. (2000) Deletion analogues of transportan. Biochim. et Biophys. Acta Biomembranes 1467, 165-76.
[17] Jones S. et al. (2010) Characterisation of bioactive cell penetrating peptides from cytochrome c: protein mimicry and the development of a novel apoptogenic agent. Chem. & Biol. 17, 735-44.
[18] Wakula P. et al. (2003) Degeneracy and function of the ubiquitous RVXF motif that mediates binding to Protein Phosphatase-1. J. Biol. Chem. 278, 18817-23.
[19] Hendrickx A et al. (2009) Docking motif-guided mapping of the interactome of Protein Phosphatase-1. Chem. & Biol. 16, 365-71.
[20] Hurley T.D. et al., (2007) Structural basis for regulation of protein phosphatase 1 by inhibitor-2. J. Biol. Chem., 282, 28874-83.
[21] Llanos S. et al. (2011) The inhibitory member of the apoptosis stimulating proteins of p53 family (iASPP) interacts with protein phosphatase (PP1) via a non-canonical binding motif. J. Biol. Chem. 286, 43039-44.
[22] Ayllon V. et al. (2002) The anti-apoptotic molecules Bcl-xL and Bcl-w target protein phosphatase 1a to Bad. Eur. J. Immunol. 32, 1847-55.
[23] Freitas M.J. et al. (2014) TCTEX1D4 interactome in human testis: Unraveling the function of dynein light chain in spermatozoa. OMICS 18, 242-53.
[24] Brush M.H., Weiser D.C. & Shenolikar S. (2003) Growth Arrest and DNA Damage-Inducible Protein GADD34 targets Protein Phosphatase 1α to the endoplasmic reticulum and promotes dephosphorylation of the α Subunit of Eukaryotic Translation Initiation Factor 2. Mol. Cell. Biol. 23, 1292-303.
[25] Ragusa M.J. et al. (2010) Spinophilin directs protein phosphatase 1 specificity by blocking substrate binding sites. Nat. Struct. Mol. Biol. 17, 459-64.
[26] Howl J. & Jones S. (2015) Protein mimicry and the design of bioactive cell penetrating peptides. In: Cell-penetrating Peptides. Methods and protocols. Second Edition (Langel, U. ed.) Springer Press, pp 177-90.
[27] Tarn J.P., Jiaxi X.J. & Eom K.D. (2001) Methods and strategies of peptide ligation. Biopolymers (Pept. Sci.) 60, 194-205.
[28] Morris J. et al. (2015) Cell penetrating peptides, targeting the regulation of store-operated channels, slow decay of the progesterone-induced [Ca2+]i signal in human sperm. Mol. Hum. Reprod. 21, 563-70.
[29] Jones S. et al. (2016) Intracellular Target-Specific Accretion of Cell Penetrating Peptides and Bioportides: Ultrastructural and Biological Correlates. Bioconjugate Chem. 27, 121-9.
[30] O'Flynn O'Brien K.L., Varghese A.C. & Agarwal A. (2010) The genetic causes of male factor infertility: a review. Fertil. Steril. 93, 1-12

## Claims

1. A peptide comprising or consisting of the following amino acid sequence:
*H*-GQQDQDR**KVICF**VAVSTLNV-*NH₂*.

2. A peptide comprising or consisting of the following amino acid sequence:
*H*-KPNA TRPVTPPRVASGLNPSIQKASNYRNNTVLY-*NH₂*.

3. A peptide according to claim 1 or 2, wherein the peptide comprises a cell-penetrating peptide.

4. A peptide according to any one of claims 1 to 3, wherein the cell-penetrating peptide is selected from the group consisting of penetratin (RQIKIWFQNRRMKWKK), HIV-derived tat (GRKKRRQRRRPQRNYF), transportan 10 (AGYLLGKINLKALAALAKKI), C105Y (CSIPPEVKFNKPFVYLI), mitoparan (INLKKLAKL(Aib)KKIL), nosangiotide (RKKTFKEVANAVKISA), cytochrome C**⁵⁻¹³** (KGKKIFIMK).

5. A peptide according to claim 4, wherein the cell-penetrating peptide is penetratin.

6. A peptide according to any one of claims 1 to 5, for use in increasing or decreasing sperm motility by modulation of the phosphoprotein phosphatase 1 complexes.

7. A peptide according to any one of claims 1 to 5, for use in modulating phosphoprotein phosphatase 1 complexes.

8. A peptide according to any one of claims 1 to 5, for use in modulating sperm motility.

9. A peptide according to any one of claims 1 to 5, for use in the treatment of male infertility.

10. A method of providing contraception, comprising administering an effective amount of a peptide according to any one of claims 1 to 5 to a subject in need of male contraception.

11. A pharmaceutical composition comprising a peptide according to any one of claims 1 to 5 and a pharmaceutically acceptable carrier.

## Patentansprüche

1. Peptid, umfassend die folgende bzw. bestehend aus der folgenden Aminosäuresequenz:
*H*-GQQDQDR**KVICF**VAVSTLNV-*NH₂*.

2. Peptid, umfassend die folgende bzw. bestehend aus der folgenden Aminosäuresequenz:
*H*-KPNATRPVTPPRVASGLNPSIQKASNYRNNTVLY-*NH₂*.

3. Peptid nach Anspruch 1 oder 2, wobei das Peptid ein zellpenetrierendes Peptid umfasst.

4. Peptid nach einem der Ansprüche 1 bis 3, wobei das zellpenetrierende Peptid ausgewählt ist aus der Gruppe bestehend aus Penetratin (RQIKIWFQNRRMKWKK), von HIV stammendem tat (GRKKRRQRRRPQRNYF), Transportan 10 (AGYLLGKINLKALAALAKKI), C105Y (CSIPPEVKFNKPFVYLI), Mitoparan (INLKKLAKL(Aib)KKIL), Nosangiotid (RKKTFKEVANAVKISA), Cytochrom C⁵⁻¹³ (KGKKIFIMK).

5. Peptid nach Anspruch 4, wobei es sich bei dem zellpenetrierenden Peptid um Penetratin handelt.

6. Peptid nach einem der Ansprüche 1 bis 5, zur Verwendung beim Erhöhen oder Verringern von Spermienmotilität durch Modulation der Phosphoprotein-Phosphatase-1-Komplexe.

7. Peptid nach einem der Ansprüche 1 bis 5, zur Verwendung beim Modulieren von Phosphoprotein-Phosphatase-1-Komplexen.

8. Peptid nach einem der Ansprüche 1 bis 5, zur Verwendung beim Modulieren von Spermienmotilität.

9. Peptid nach einem der Ansprüche 1 bis 5, zur Verwendung bei der Behandlung von männlicher Infertilität.

10. Verfahren zur Bereitstellung von Kontrazeption, umfassend Verabreichen einer wirksamen Menge eines Peptids nach einem der Ansprüche 1 bis 5 an ein Individuum, das männliche Kontrazeption benötigt.

11. Pharmazeutische Zusammensetzung, umfassend ein Peptid nach einem der Ansprüche 1 bis 5 und einen pharmazeutisch unbedenklichen Träger.

## Revendications

1. Peptide comprenant ou constitué des la séquence d'acides aminés suivante :
*H*-GQQDQDR**KVICF**VAVSTLNV-*NH₂*.

2. Peptide comprenant ou constitué de la séquence d'acides aminés suivante :
*H*-KPNATRPVTPPRVASGLNPSIQKASNYRNNTVLY-*NH₂*.

3. Peptide selon la revendication 1 ou 2, où le peptide comprend un peptide de pénétration cellulaire.

4. Peptide selon l'une quelconque des revendications 1 à 3, dans lequel le peptide de pénétration cellulaire est choisi dans le groupe constitué de : la pénétratine (RQIKIWFQNRRMKWKK), tat dérivé du VIH (GRKKRRQRRRPQRNYF), le transportan 10 (AGYLLGKINLKALAALAKKI), C105Y (CSIPPEVKFNKPFVYLI), le mitoparan (INLKKLAKL(Aib)KKIL), le nosangiotide (RKKTFKEVANAVKISA), le cytochrome C⁵⁻¹³ (KGKKIFIMK) .

5. Peptide selon la revendication 4, dans lequel le peptide de pénétration cellulaire est la pénétratine.

6. Peptide selon l'une quelconque des revendications 1 à 5, pour utilisation dans l'augmentation ou la diminution de la motilité des spermatozoïdes par modulation des complexes de phosphoprotéine phosphatase 1.

7. Peptide selon l'une quelconque des revendications 1 à 5, pour utilisation dans la modulation de complexes de phosphoprotéine phosphatase 1.

8. Peptide selon l'une quelconque des revendications 1 à 5, pour utilisation dans la motilité des spermatozoïdes.

9. Peptide selon l'une quelconque des revendications 1 à 5, pour utilisation dans le traitement de l'infertilité masculine.

10. Procédé de fourniture d'une contraception, comprenant l'administration d'une quantité efficace d'un peptide selon l'une quelconque des revendications 1 à 5 à un sujet ayant besoin d'une contraception masculine.

11. Composition pharmaceutique comprenant un peptide selon l'une quelconque des revendications 1 à 5 et un véhicule pharmaceutiquement acceptable.
